Europäisches Patentamt

**European Patent Office**

**Office européen des brevets**

(19)

(11) Veröffentlichungsnummer : **0 030 722**
**B1**

(12)

# EUROPÄISCHE PATENTSCHRIFT

(45) Veröffentlichungstag der Patentschrift :
26.09.84

(21) Anmeldenummer : **80107858.5**

(22) Anmeldetag : **12.12.80**

(51) Int. Cl.³ : **A 61 B 5/05**, A 61 B 5/08,
G 01 R 27/02

(54) **Messvorrichtung zur Thoraximpedanzmessung.**

(30) Priorität : **12.12.79 DE 2949887**

(43) Veröffentlichungstag der Anmeldung :
**24.06.81 Patentblatt 81/25**

(45) Bekanntmachung des Hinweises auf die Patenterteilung : **26.09.84 Patentblatt 84/39**

(84) Benannte Vertragsstaaten :
**BE CH DE FR GB IT LI NL SE**

(56) Entgegenhaltungen :
**FR-A- 1 575 190**
**FR-A- 2 020 493**
**FR-A- 2 239 974**
**FR-A- 2 345 980**
**US-A- 3 871 359**

(73) Patentinhaber : **Dankwart, Franz-Josef, Dipl.-Phys.**
**Nienkamp 4**
**D-4400 Münster (DE)**

**Freitag, Lutz**
**An der Altenburg 28**
**D-4300 Essen (DE)**

(72) Erfinder : **Dankwart, Franz-Josef, Dipl.-Phys.**
**Nienkamp 4**
**D-4400 Münster (DE)**
Erfinder : **Freitag, Lutz**
**An der Altenburg 28**
**D-4300 Essen (DE)**

(74) Vertreter : **Von Bezold, Dieter et al**
**v.Bezold, Dr. Dieter Schütz, Peter, Dipl.-Ing. Heusler,**
**Wolfgang, Dipl.-Ing. Patentanwälte**
**Postfach 86 02 60 D-8000 München 86 (DE)**

**Beschreibung**

Die Erfindung betrifft eine Meßvorrichtung zur Thoraximpedanzmessung, bei der

a) die Thoraximpedanz mit Hilfe von durch einen Wechselstromgenerator gespeisten Auflageelektroden mit eingeprägtem, konstantem Strom durch Spannungsmessung bestimmt wird,

b) die Meßvorrichtung einen linken (L) und einen rechten (R) Verstärkerkanal aufweist,

c) in den Verstärkerkanälen (L, R) phasensteuerbare Gleichrichter (13) vorgesehen sind, und

d) die Ausgänge dieser Gleichrichter (13) mit zugeordneten Spannungsanzeigegeräten (15) verbunden sind.

Eine solche Meßvorrichtung ist aus US-A 3,871,359 bekannt. Bei dieser bekannten Meßvorrichtung sind der Wechselstromgenerator und zwei zu messende biologische Impedanzen in einem Stromkreis in Reihe geschaltet, wobei die Impedanzen jeweils über ein Paar von Auflageelektroden angeschlossen sind. Von jeder Impedanz wird durch zwei weitere, eigene Auflageelektroden, die sich zwischen den im Stromkreis des Wechselstromgenerators geschalteten Elektroden befinden, eine Spannung abgegriffen. Die abgegriffenen Spannungen können jeweils durch einen phasengesteuerten Gleichrichter gleichgerichtet werden, um ein dem Realteil und ein dem Imaginärteil der gemessenen Impedanz entsprechendes Signal zu erzeugen.

Als Impedanz wird der komplexe Wechselstromwiderstand $Z_0$ bezeichnet, der in biologischen Geweben im wesentlichen durch die relativ konstante Ionenkonzentration bestimmt ist.

In der Zeitschrift CHEST, Vol. 64, Nr. 5, Nov. 1973, S. 597 wird auch bereits darauf hingewiesen, daß mit Hilfe einer derartigen Impedanzmessung transthorakale Impedanzmessungen mit Wechselströmen von 100 KHz und 0,3 mA sowie 60 KHz und 0,5 mA in einem Dreipolelektrodensystem ausgeführt worden sind. Derartige Impedanzmessungen dienen der Überwachung der Lungenfunktion und zeigen insbesondere eine Abnahme des Luftvolumens oder eine Ansammlung von Flüssigkeiten als Impedanzabfall an. Aus der gleichen Literaturstelle ist es auch bekannt, mit Hilfe von Impedanzmessungen das Herzschlagvolumen zu bestimmen. Dabei wurde ein Wechselstrom von 100 KHz und 4 mA in Verbindung mit einem digitalanzeigenden Voltmeter benutzt.

Es ist ferner aus der Literaturstelle « Transthoracic Electrical Impedance » American Heart Journal Vol. 85, 1, S. 83-93, ein Verfahren zur Messung des Lungenzustandes durch Impedanzmessung bekannt, bei dem jedoch keine Trennung der beiden Lungenseiten und keine angenäherte Realteilmessung von $Z_0$, sondern nur eine Messung des komplexen $Z_0$-Wertes vorgesehen ist.

Allgemeine Grundlagen der Impedanzmessung sind ferner in dem Buch « Impedanz-Kardiographie » E. Lang, R. Kessel und A. Winkl Verlag Christian M. Silinsky 1978, dargestellt.

Ein Pneumograph mit getrennter Impedanzmessung beider Lungenseiten ist in der Literaturstelle « Electronic Techniques in Anaesthesia and Surgery » Hill 1973, S. 123, beschrieben. Hier wurden für beide Lungenseiten streifenförmige Meßelektroden und verschiedene Meßfrequenzen (50 KHz und 20 KHz) verwendet. Es kann keine $Z_0$-Messung durchgeführt werden, da sich aus den verschiedenen Imaginärteilen erhebliche Meßfehler ergeben würden.

Die vorbekannten Verfahren und Meßvorrichtungen ergeben, insbesondere dann, wenn beide Lungenteile getrennt überwacht werden sollen, keine hinreichende Genauigkeit der Thoraximpedanzmessung. Bei den bekannten Meßvorrichtungen und den dabei verwendeten Frequenzen traten nämlich in der komplexen Größe der Impedanz Imaginärteilfehler bis zu 30 % auf.

Die Erfindung geht von der Aufgabenstellung aus, eine Meßvorrichtung der eingangs beschriebenen Art zu schaffen, welche genaue Messungen wesentlicher Bestimmungsgrößen für beide Lungenteile ermöglicht und die außerdem durch die Art der Meßwertbildung eine gegenseitige Kompensation von pathologischen Abläufen im Meßergebnis ausschließt.

Die Lösung dieser Aufgabe besteht darin, daß bei einer Meßvorrichtung zur Thoraximpedanzmessung mit den eingangs aufgeführten Merkmalen a) bis d) erfindungsgemäß

e) die Auflageelektroden mindestens eine kraniale Elektrode, die mit beiden Verstärkerkanälen (L, R) verbunden ist und zwei Thoraxseitenelektroden, welche mit dem rechten bzw. dem linken Verstärkerkanal (L, R) verbunden sind, umfassen,

f) daß zur Kanaltrennung ein Phasenschieber vorgesehen ist, durch den der Konstantstrom für den einen Kanal gegenüber dem Konstantstrom für den anderen Kanal um 90° in der Phase verschoben wird ;

g) daß die phasensteuerbaren Gleichrichter entsprechend den. Phasen der Konstantströme des rechten bzw. linken Kanals gesteuert sind, und

h) die Ausgänge der Gleichrichter jeweils unter Zwischenschaltung eines Integrators mit dem zugeordneten Spannungsanzeigegerät verbunden sind.

Durch die Verwendung einer einzigen Meßfrequenz und die Kanaltrennung durch Phasenunterscheidung lassen sich bei einer Meßfrequenz, die zweckmäßig im Tonfrequenzbereich zwischen 5 und 15 KHz, vorzugsweise bei 10 KHz liegt, Meßfehler der Grundimpedanz unterhalb von 3 % erreichen. Der Meßstrom kann dabei nur 0,3 $mA_{eff}$ betragen, so daß ein Ansprechen sensibler oder motorischer Nerven mit Sicherheit ausgeschlossen wird. Wesentlich ist die Verwendung der mit beiden Kanälen verbundenen Integratoren, welche die Thoraximpedanz, z. B. vorteilhafterweise wahlweise mit einer Zeitkonstante von 0,1 ; 1,0 oder 10 s, integrieren. Die integrierten Signale werden dann durch ein zugehöriges Spannungszeigegerät digital angezeigt.

Die Messung der Thoraxgrundimpedanz $Z_0$ als Augenblickswert in Expiration wie üblich zur Standardisierung der Meßmethode ist erstens oft nicht möglich (z. B. bei tachypneumischen Patienten) und zweitens nicht sinnvoll bei beatmeten Patienten, da die Beatmung, insbesondere die Peep-Beatmung, einen erwünschten Effekt auf das (Luft-) Residualvolumen und den Lungenflüssigkeitsgehalt hat. Um diesen erwünschten Effekt zu verifizieren und Artefakte durch stockende Atmung, Husten oder Thoraxbewegungen zu unterdrücken, wird eine entsprechende Meßwertintegration, sinnvollerweise mit einer Zeitkonstanten von 10 s benutzt. Hierbei ist der $Z_0$-Betrag medizinisch neu zu definieren. Um Vergleiche mit anderen Meßwertgewinnungen zu ermöglichen, sind Messungen mit den anderen Zeitkonstanten möglich.

Die Meßvorrichtung mißt die Impedanz $Z_0$ der rechten und linken Lunge getrennt und ebenso die atmungsbedingten Änderungen $\Delta Z$ der beiden Lungenseiten. Auf diese Weise ist eine weitgehende Differenzierung zwischen akut auftretenden luft- und wasserbedingten Impedanzänderungen möglich, wenn man in die Überlegung einbezieht, welche Prozesse beidseitig und welche vorwiegend einseitig auftreten. So ergibt sich folgendes Logikschema für Thorax-Lungenprozesse : Fluidlunge → beide $Z_0$ — Werte sinken ; Pneumothorax → $Z_0$ dieser Pneuseite steigt ; $\Delta Z$ fällt ; Pleuraerguß → $\Delta Z$ fällt ebenfalls ab, aber $Z_0$ fällt auch, usw.

Es kann ferner zweckmäßig sein, den gleichrichtern in den Kanälen Bandfilter mit einem Durchlaßbereich zwischen $f_u = 300$ Hz und $f_0 = 25$ KHz zur Unterdrückung der EKG-Signals und zur Phasenkorrektur vorzuschalten. Schaltungstechnisch zweckmäßig erscheint die Verwendung eines Tonfrequenzgenerators, welcher mit dem einen Stromkonstanthalter direkt und mit dem anderen Stromkonstanthalter unter Zwischenschaltung des 90° — Phasenschiebers verbunden ist, wobei Rechteckformer vorgesehen sind, welche Rechteckspannungen in der Phasenlage des direkt angeschlossenen Stromkonstanthalters und in der um 90° verschobenen Phasenlage erzeugen und bei der die beiden Rechteckspannungen den phasensteuerbaren Gleichrichtern in den Kanälen als Steuersignal zugeführt werden.

In weiterer Ausbildung der Erfindung kann es zweckmäßig sein, daß ein erster Analogaddierer mit den beiden Kanälen am Ein- und Ausgang der bandfilter verbunden ist, dessen Ausgang unter Zwischenschaltung eines nach den EKG-Frequenzen ausgerichteten Tiefpasses mit einem zusätzlichen Impedanzkardiogramm- oder IKG-Teil in Verbindung steht sowie die Überwachung des EKG's mit einem handelsüblichen EKG-Monitor zuläßt, ohne weitere Elektroden aufbringen zu müssen. Durch den IKG-Teil können mit der gleichen Impedanzmessung sowohl der Lungenzustand als auch kardiale Bewertungsgrößen, näherungsweise das Herzzeitvolumen bestimmt werden. Die herzschlagbedingten Impedanzänderungen werden dabei zweckmäßig durch mehrpolige Bandfilter ausgefiltert.

Eine gegebenenfalls zweckmäßige Weiterbildung kann vorsehen, daß der IKG-Teil einen Zweiten Analogaddierer enthält, welcher mit den gleichspannungsausgängen der beiden phasengesteuerten Gleichrichter in den Kanälen verbunden ist und daß der Ausgang des zweiten. Analogaddierers mit einem. Differenzierer verbunden ist. Zur Auswertung des dZ/dt-Signals kann vorteilhaft dem zweiten. Differenzierer ein Peakdetektor mit einstellbarem Nullpunkt nachgeschaltet sein, welcher entweder intern von einem dem EKG-Filter nachgeschalteten ersten. Differenzierer, einem Impulsformer und einem Monoflop aus der R-Zacke des EKG-Signals oder extern triggerbar ist und somit die Spannungsdifferenz zwischen der Spannung zum Triggerzeitpunkt $t_n$ und der maximalen Ausgangsspannung zwischen zwei Triggerzeitpunkten $t_n$ und $t_{n+1}$ bildet. Dabei kann vorteilhaft zwischen den zweiten Addierer und den zweiten Differenzierer, welcher eine Grenzfrequenz von 50 Hz aufweist, ein Bandfilter mit einem Frequenzbereich $f_u = 0,5$ Hz und $f_0 = 50$ Hz zwischengeschaltet werden, welches ebenfalls als mehrpoliges Bandfilter ausgebildet sein soll und den herzspezifischen Frequenzbereich abtrennt.

Die Elektroden sind zweckmäßig als Gel-beschichtbare Doppelelektroden ausgebildet, wobei die Thoraxseiten-Elektroden auf die linke und rechte Thoraxseite etwa in Höhe des fünften Interkostalraumes aufgeklebt werden. Die kraniale Elektrode kann gegebenenfalls auch durch zwei seitlich versetzte Elektroden ersetzt werden. Damit läßt sich unter Umständen eine bessere Anpassung an bereits bekannte lokale Herde erzielen.

Durch die angegebene Meßvorrichtung zur Thoraximpedanzmessung ist die gleichzeitige Messung der über einen Zeitraum gemittelten Thoraxgrundimpedanz der rechten und linken Körperseite, und zwar nahezu imaginärfrei möglich. Dabei kann eine gleichzeitige Darstellung der Atemtiefen beider Lungen erreicht werden. Bei Ausfilterung der herzspezifischen Frequenz läßt sich eine gleichzeitige Herzschlagvolumenmessung durchführen.

In der Zeichnung ist ein bevorzugtes Ausführungsbeispiel des Gegenstandes der Erfindung schematisch dargestellt ; es zeigen :

Figur 1 eine Anschlußskizze der Meßvorrichtung in verbindung mit den Elektroden,

Figur 2 ein Blockschaltbild mit beiden Verstärkerkanälen,

Figur 3 ein Blockschaltbild, welches den Wechselstromgenerator und die Stromkonstanthalter zur Verbindung mit den Einheiten der Schaltbilder aus Fig. 1 und 2 enthält,

Figur 4 einen IKG-Teil zur Verbindung mit dem Blockschaltbild nach Fig. 2.

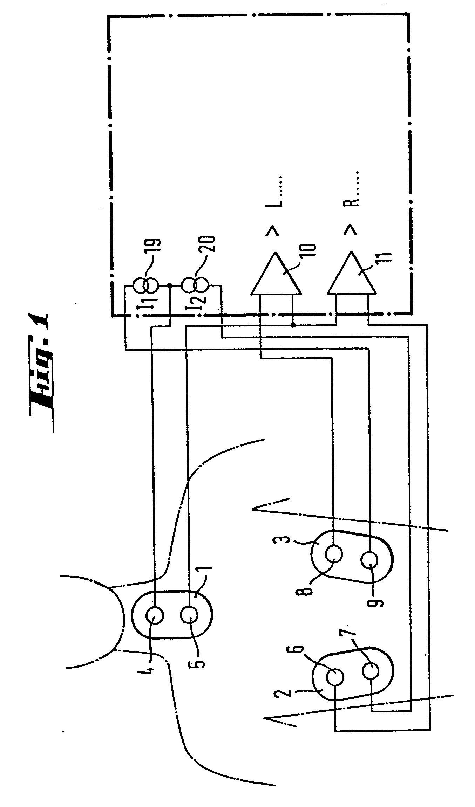

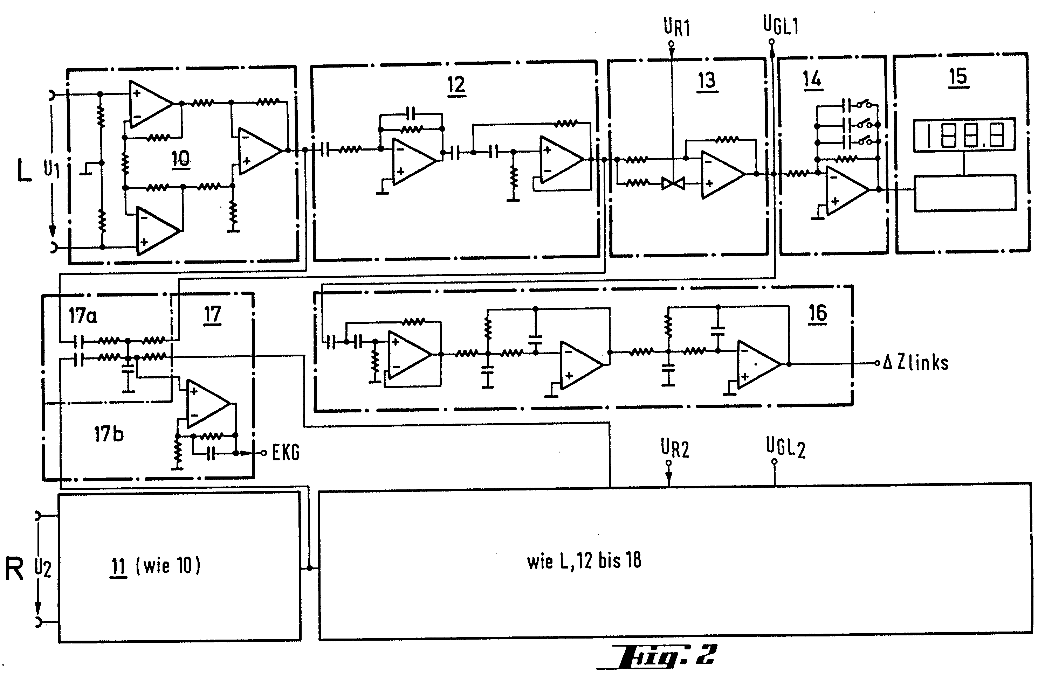

In Fig. 1 sind eine kraniale Elektrode 1 und zwei Thoraxseitenelektroden 2, 3 dargestellt, an denen jeweils zwei elektrisch getrennte Kontaktanschlußflächen 4, 5 ; 6, 7 bzw. 8, 9 vorgesehen sind, welche wie dargestellt über Leitungen mit einem linken bzw. rechten Verstärkerkanal L bzw R sowie mit zuge ordneten Stromkonstanthaltern 19 bzw. 20 verbunden sind. Die näheren schaltungstechnischen Einzelheiten ergeben sich aus den Blockschaltbildern in Fig. 2, 3 und 4.

Wie in Fig. 2 gezeigt, enthalten die beiden Kanäle L und R je einen Differenz-Vorverstärker 10 bzw. 11. Der sich an die Vorverstärker anschließende Schaltungsteil ist nur für den linken Kanal L genauer dargestellt, der rechte Kanal ist entsprechend geschaltet. Auf den Vorverstärker 10 folgt ein Bandfilter 12 mit einer unteren Grenz- frequenz $f_u = 300$ Hz (dreipolig) und einer oberen Grenzfrequenz $f_0 = 25$ kHz (einpolig) zur EKG-Unterdrückung und Phasenkorrektur.

Vom Bandfilter 12 wird das Signal an einen phasensteuerbaren Gleichrichter 13 weitergeleitet, an dessen Ausgang ein Integrator 14 mit einstellbarer Zeitkonstante angeschlossen ist. Der Ausgang des Integrators ist mit einem digitalen Spannungsanzeigegerät 15 gekoppelt. Der Ausgang der phasensteuerbaren Gleichrichter 13 ist ferner mit einem mehrpoligen Bandfilter 16, dessen Durchlaßbereich $f_u = 0,05$ Hz bis $f_0 = 1$ Hz beträgt, verbunden, welches die lungenspezifischen Impedanzänderungen ausfiltert.

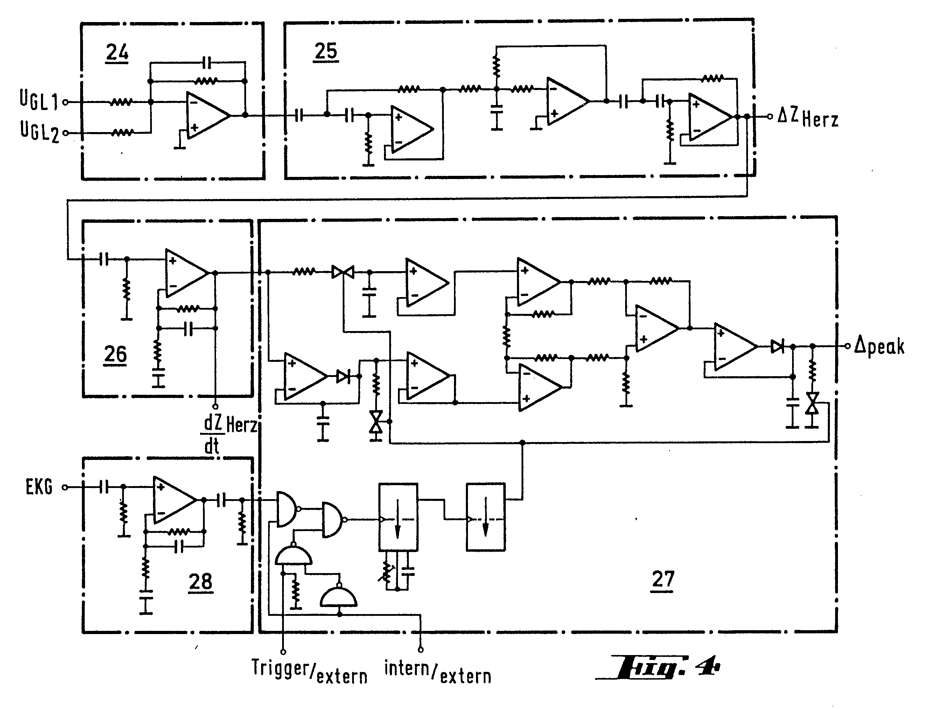

Zum Ausschluß eines EKG-Teils ist ein erster Analogaddierer 17a mit Tiefpaß mit den Ein- und Ausgängen der beiden Bandfilter 12 verbunden. Sein Ausgang führt über einen weiteren Tiefpaß 17b zum Eingang eines EKG-Meß- und Anzeigeteils, der anhand des Schaltbildes gemäß Fig. 4 erläutert wird. Der erste Analogaddierer 17a bildet mit dem nachfolgenden Tiefpaß 17b ein EKG-Filter mit einem Durchlaßbereich von z. B. 0,5 bis 50 Hz. Das Ausgangssignal des EKG- Filters kann über einen Spannungsteiler einem EKG-Monitor zugeführt werden.

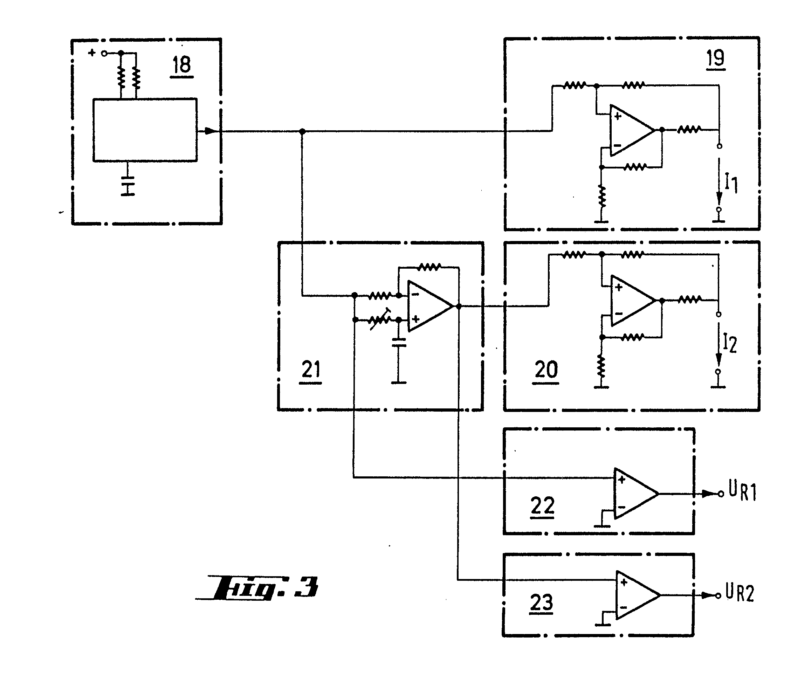

Die Stromversorgung gemäß Fig. 3 enthält einen 10-kHz-Generator 18 mit nachgeschalte- tem, nicht besonders dargestellten Trennver- stärker oder Buffer, welcher mit einem Stromkonstanthalter 19 direkt und mit einem Stromkonstanthalter 20 unter Zwischenschlatung eines Phasenschiebers 21 verbunden ist, der bei der Generatorfrequenz auf eine Phasenverschiebung von exakt 90° einstellbar ist. Die Wechselspannungen des Generators 18 und des Phasenschiebers 21 werden durch Rechteckformer 22, 23 in Rechtecksignale umgeformt, die in der Phasenlage entsprechend den Strömen um 90° gegeneinander verschoben sind. Diese Rechtecksignale dienen zur Steuerung des Durchlasses der phasensteuerbaren Gleichrichter 13 (in Fig. 2).

Die Schaltung gemäß Fig. 4 enthält einen ersten, an das EKG-Filter 17 angeschlossenen Differenzierer 28 und Impulsformer sowie einen Monoflop zum Erzeugen des Triggersignals durch die R-Zacke. Sein Ausgang führt zu einem Peakdetektor 27 mit einstellbarem Nullpunkt, wobei als Nullpunkt die Spannung angenommen wird, die zum Triggerzeitpunkt zuzüglich der Verzögerungszeit durch den einstellbaren Monoflop am Peakdetektor anliegt. Die Triggerung kann auch extern über den ersten Differenzierer 28 erfolgen.

Der Peakdetektor 27 wird von einem zweiten Differenzierer 26 mit dem Anstiegsignal dZ/dt gespeist. Dem zweiten Differenzierer 26, der eine Grenzfrequenz von 50 Hz aufweist, sind ein mehrpoliges Bandfilter 25 mit einem Durchlaßbereich von $f_u = 0,5$ Hz bis $f_0 = 50$ Hz und eine Addierer- und Tiefpaßschaltung 24 vorgeschaltet. Am Ausgang des Bandfilters 25 kann ein Signal $dZ_{Herz}/dt$, das der herzspezifischen Impe- danzänderung entspricht, abgenommen werden. Die Addierer- und Tiefpaßschaltung 24 wird eingangsseitig mit den Ausgangsgleichspannungen $U_{GL1}$ und $U_{GL2}$ der phasensteuerbaren Gleich- richter 13 gespeist.

Die Wirkungsweise der Schaltung kann wie folgt beschrieben werden : In den Stromkreis der Doppelektroden 1, 2 und 3 werden konstante, um 90° phasenverschobene Ströme $I_1$ und $I_2$ durch den Wechselstromgenerator 18 und die Stromkonstanthalter 19 bzw. 20 eingespeist. Die abgegriffenen Spannungen $U_1$ und $U_2$ werden von den Differenz-Vorverstärkern 10, 11 des linken bzw. rechten Kanals verstärkt und die Bandfilter 12 lassen die Meßfrequenz mit den entsprechenden Seitenbändern durch, während das EKG-Signal unterdrückt wird. Die Nachgeschalteten phasensteuerbaren Gleichrichter 13 (« Synchron-Demodulatoren ») geben eine Ausgangsspannung an die Integratoren 14 und an die nachfolgenden Digitalvoltmeter 15 nur dann ab, wenn die der Kanaltrennung entsprechende Phasenlage vorliegt. Hierzu werden die phasensteuerbaren Gleichrichter 13 durch entsprechende, um 90° verschobene Steuerspannungen $U_{R1}$ und $U_{R2}$ von den Rechteckformen 22 bzw. 23 gesteuert.

Die Ausgangssignale der Differenzverstärker 10, 11 werden außerdem dem Eingang des ersten Analogaddierers 17a zugeführt, aus dessen Ausgangssignal durch das EKG-Filter 17a, b nunmehr die für das EKG-Signal wesentlichen Frequenzen ausgefiltert werden. Vom Ausgang des EKG- Filters 17 kann das EKG-Signal über einen Spannungsteiler einem EKG-Monitor z. B. einem Oszillographen, zugeführt werden. Das EKG- Signal wird ferner dem ersten Differenzierer 28 zugeführt, welcher mit einem Impulsformer ein Triggersignal aus dem Anstieg der R-Zacke des EKG-Signales bildet. Die Ausgangssignale der phasensteuerbaren Gleichrichter 13 sind Gleichspannungen $U_{GL1}$ und $U_{GL2}$, die den Eingängen des Addierers 24 zugeführt werden. Sein Ausgangssignal wird durch das steilflankige Bandfilter 30 auf Frequenzen zwischen 0,5 und 50 Hz gefiltert, so daß einerseits eine herzspezifische Änderungsgröße $\Delta Z$ und andererseits nach Wei- terverarbeitung des Signals im zweiten Differenzierer 26, eine Signalgröße, welche der Änd- ungsgeschwindigkeit des $\Delta Z$-Signals entspricht, zur Verfügung stehen. Dieses Änderungssignal wird dem Peakdetektor 27 mit einstellbarem Nullpunkt zugeführt, welcher entweder extern oder durch das Ausganssignal des ersten Differenzierers 28 getriggert wird. Die Ausgangsgröße des Peakdetektors 27 wird zur Bestimmung des Herzschlagvolumens verwendet. Die R-Zacke des EKG-Signales wird geräteintern als Triggerpunkt für die dz/dt-Analyse verwendet. Aus der Änderungsgeschwindigkeit der Impedanz im herztypischen Frequenzbereich kann das Herzschlagvolumen annähernd ermittelt werden.

**Ansprüche**

1. Meßvorrichtung zur Thoraximpedanzmessung, bei der
a) die Thoraximpedanz mit Hilfe von durch einen Wechselstromgenerator gespeisten Auflageelektroden mit eingeprägtem, konstantem Strom durch Spannungsmessung bestimmt wird,
b) die Meßvorrichtung einen linken (L) und einen rechten (R) Verstärkerkanal aufweist,
c) in den Verstärkerkanälen (L, R) phasensteuerbare Gleichrichter (13) vorgesehen sind, und
d) die Ausgänge dieser Gleichrichter (13) mit zugeordneten Spannungsanzeigegeräten (15) verbunden sind,
dadurch gekennzeichnet, daß
e) die Auflageelektroden mindestens eine kraniale Elektrode (1), die mit beiden Verstärkerkanälen (L, R) verbunden ist und zwei Thoraxseitenelektroden (2, 3), welche mit dem rechten bzw. dem linken Verstärkerkanal (L, R) verbunden sind, umfassen,
f) daß zur Kanaltrennung ein Phasenschieber (21) vorgesehen ist, durch den der Konstantstrom für den einen Kanal gegenüber dem Konstantstrom für den anderen Kanal um 90° in der Phase verschoben wird ;
g) daß die phasensteuerbaren Gleichrichter (13) entsprechend den Phasen der Konstantströme des rechten bzw. linken Kanals gesteuert sind, und
h) die Ausgänge der Gleichrichter (13) jeweils unter Zwischenschaltung eines Integrators (14) mit dem zugeordneten Spannungsanzeigegerät (15) verbunden sind.

2. Meßvorrichtung nach Anspruch 1, dadurch gekennzeichnet, daß den Gleichrichtern (13) in den Kanälen (L, R) jeweils ein Bandfilter (12) mit einem Durchlaßbereich zwischen $f_u = 300$ Hz und $f_0 = 25$ kHz zur Unterdrückung des EKG-signals und zur Phasenkorrektur vorgeschaltet ist.

3. Meßvorrichtung nach Anspruch 1, dadurch gekennzeichnet, daß der gemeinsame Wechselstromgenerator (18) ein Tonfrequenzgenerator mit einer Frequenz zwischen 5 und 15 kHz, vorzugsweise 10 kHz ist.

4. Meßvorrichtung nach Anspruch 1, 2 und 3, dadurch gekennzeichnet, daß der Wechselstromgenerator (18) mit einem ersten Stromkonstanthalter (19) direkt und mit einem zweiten Stromkonstanthalter (20) unter Zwischenschaltung des 90°-Phasenschiebers (21) verbunden ist, daß die Stromkonstanthalter (19, 20) jeweils mit einem Rechteckformer (22, 23) gekoppelt sind, welcher Rechteckspannungen in der Phasenlage des direkt angeschlossenen Stromkonstanthalters (19) bzw. in der um 90° verschobenen Phasenlage erzeugt, und daß die beiden Rechteckspannungen den phasensteuerbaren Gleichrichtern (13) in den Kanälen (L, R) als Steuersignal zugeführt sind.

5. Meßvorrichtung nach Anspruch 4, dadurch gekennzeichnet, daß ein erster Analogaddierer (17a) mit den beiden Kanälen (L, R) am Ein- und Ausgang der Bandfilter (12) verbunden ist, dessen Ausgang unter Zwischenschaltung eines die EKG-Frequenzen selektierenden Tiefpasses (17a, b) mit einem zusätzlichen Impedanzkardiogramm (IKG)-Teil in Verbindung steht sowie das EKG-Signal über die Auflageelektroden (1, 2, 3) ableitet.

6. Meßvorrichtung nach Anspruch 5, dadurch gekennzeichnet, daß der IKG-Teil einen zweiten Analogaddierer (24) enthält, welcher mit den Gleichspannungsausgängen der beiden phasengesteuerten Gleichrichtern (13) in den Kanälen (L, R) verbunden ist, daß der Ausgang des zweiten Analogaddierers (24) über ein steilflankiges Bandfilter (25), das einen Durchlaßfrequenzbereich entsprechend den herzspezifischen Impedanzänderungen von $f_u = 0,5$ Hz bis $f_o = 50$ Hz hat, mit einem zweiten Differenzierer (26) verbunden ist.

7. Meßvorrichtung nach Anspruch 6, dadurch gekennzeichnet, daß dem zweiten Differenzierer (26) ein Peakdetektor mit einstellbarem Nullpunkt nachgeschaltet ist, der die Differenz zwischen der maximalen Ausgangsspannung des zweiten Differenzierers (26) zwischen zwei Triggerzeitpunkten $t_n$ und $t_{n+1}$ und der Ausgangsspannung zum Triggerzeitpunkt $t_n$ bildet, der entweder intern über einen ersten Differenzierer (28) und einen nachgeschalteten Impulsformer aus der R-Zacke des EKG-Signals, oder extern erzeugt ist.

8. Meßvorrichtung nach Anspruch 1, dadurch gekennzeichnet, daß die Anflageelektroden (1, 2, 3) Gel-beschichtbare Doppelelektroden sind.

**Claims**

1. A measuring apparatus for thorax impedance measurement, wherein :
a) the thorax impedance is determined by voltage measurement by means of contact electrodes with an impressed constant current, the electrodes being supplied by an alternating current generator,
b) the measuring apparatus has a left (L) and a right (R) amplifier channel,
c) phase-controllable rectifiers (13) are provided in the amplifier channels (L, R), and
d) the outputs of said rectifiers (13) are connected to associated voltage display devices (15), characterised in that

e) the contact electrodes comprise at least one cranial electrode (1) which is connected to both amplifier channels (L, R) and two thorax side electrodes (2, 3) which are connected to the right and the left amplifier channels (L, R) respectively,

f) for channel separation, there is provided a phase shift means (21) by means of which the constant current for the one channel is displaced through 90° in phase relative to the constant current for the other channel,

g) the phase-controllable rectifiers (13) are controlled in accordance with the phases of the constant currents of the right and the left channels respectively, and

h) the outputs of the rectifiers (13) are respectively connected to the associated voltage display device (15) by way of a respective interposed integrator (14).

2. A measuring apparatus according to claim 1, characterised in that connected on the input side of the respective rectifiers (13) in the channels (L, R) is a band filter (12) with a pass band between $f_u = 300$ Hz and $f_0 = 25$ Hz for suppressing the EKG-signal and for phase correction.

3. A measuring apparatus according to claim 1, characterised in that the common alternating current generator (18) is an audio frequency generator with a frequency between 5 and 15 kHz, preferably 10 kHz.

4. A measuring apparatus according to claim 1, claim 2 and claim 3, characterised in that the alternating current generator (18) is connected to a first means (19) for keeping the current constant directly and to a second means (20) for keeping the current constant by way of an interposed 90°-phase shift means (21), that the means (19, 20) for keeping the current constant are each respectively coupled to a square or rectangular shaper (22, 23) which produce square or rectangular voltages in the phase position of the directly connected means (19) for keeping the current constant and in the phase position which is displaced through 90°, and that the two square or rectangular voltages are fed to the phase-controllable rectifiers (13) in the channels (L, R) as a control signal.

5. A measuring apparatus according to claim 4, characterised in that a first analog adder (17a) is connected to the two channels (L, R) at the input and output of the band filters (12), the output thereof being connected to an additional impendance cardiogram (IKG)-means, by way of an interposed low pass filter (17a, 17b) which selects the EKG-frequencies, and deriving the EKG-signal by way of the contact electrodes (1, 2, 3).

6. A measuring apparatus according to claim 5, characterised in that the IKG-means includes a second analog adder (24) which is connected to the d. c. voltage outputs of the two phase-controlled rectifiers (13) in the channels (L, R), and that the output of the second analog adder (24) is connected to a second differentiator (26) by way of a steep-edge band pass filter (25) which has a pass band corresponding to the heart-specific changes in impedance of $f_u = 0.5$ Hz to $f_o = 50$ Hz.

7. A measuring apparatus according to claim 6, characterised in that connected to the output side of the second differentiator (26) is a peak detector with an adjustable zero point, which forms the difference between the maximum output voltage of the second differentiator (26) between two trigger times $t_n$ and $t_{n+1}$ and the output voltage at the trigger time $t_n$, which is either produced internally by way of a first differentiator (28) and a pulse shaper on the output side thereof, from the R-spike of the EKG-signal, or externally.

8. A measuring apparatus according to claim 1, characterised in that the contact electrodes (1, 2, 3) are gel-coatable double electrodes.

**Revendications**

1. Appareil de mesure de l'impédance du thorax dans lequel :

a) L'impédance thoracique est déterminée par mesure de la tension, à l'aide d'électrodes appliquées alimentées en courant constant à l'aide d'un générateur de courant alternatif.

b) Le dispositif de mesure comporte un canal amplificateur de gauche (L) et un canal amplificateur de droite (R).

c) On prévoit dans les canaux amplificateurs (L, R) des redresseurs (13) pouvant être commandés en phases, et

d) Les sorties de ces redresseurs (13) sont reliées à des appareils d'indication de tension (15) associés,
caractérisé par le fait que :

e) Les électrodes appliquées comportent au moins une électrode crânienne (1) reliée aux deux canaux amplificateurs (L, R) et deux électrodes thoraciques latérales (2, 3) qui sont reliées respectivement au canal amplificateur droit et au canal amplificateur gauche (L, R).

f) On prévoit pour séparer les canaux un déphaseur (21) à l'aide duquel le courant constant pour l'un des canaux est déphasé de 90° par rapport au courant constant pour l'autre canal.

g) Les redresseurs (13) pouvant être commandés en phases sont commandés en fonction des phases des courants constants du canal de droite et du canal de gauche ; et

h) Les sorties des redresseurs (13) sont reliées à l'appareil indicateur de tension (15) associé, en intercalant chaque fois un intégrateur (14).

2. Appareil de mesure selon la revendication 1, caractérisé par le fait qu'on monte avant les redresseurs (13), dans chacun des canaux (L, R) un filtre passe-bande (12) ayant une bande passante comprise entre $f_u = 300$ Hz et $f_0 = 25$ kHz pour supprimer le signal EKG (électrocardiogramme) et pour la correction de phase.

3. Appareil de mesure delon la revendication 1, caractérisé par le fait que le générateur de courant alternatif commun (18) est un générateur d'audiofréquences ayant une fréquence comprise entre 5 et 15 kHz, de préférence 10 kHz.

4. Appareil de mesure selon les revendications 1, 2 et 3, caractérisé par le fait que le générateur de courant alternatif (18) est relié directement à un premier stabilisateur de courant (19) et en intercalant le déphaseur de 90° (21) à un second stabilisateur de courant (20), que les stabilisateurs de courant (19, 20) sont couplés respectivement à un dispositif conformateur d'ondes rectangulaires (22, 23), qui produit des tensions rectangulaires dans la phase du stabilisateur de courant directement raccordé (19) ou dans la phase décalée de 90°, et que les deux tensions rectangulaires sont amenées en tant que signal de commande aux redresseurs (13) commandés en phases, dans les canaux (L, R).

5. Appareil de mesure selon la revendication 4, caractérisé par le fait qu'un premier additionneur analogique (17a) est relié aux deux canaux (L, R) à l'entrée et à la sortie des filtres passe-bandes (12) additionneur dont la sortie est reliée, en intercalant un filtre passe-bas (17a, 17b) sélectionnant les fréquences EKG à une unité de cardiogramme d'impédance supplémentaire (IKG) et dérive le signal EKG par l'intermédiaire des électrodes appliquées (1, 2, 3).

6. Appareil de mesure selon la revendication 5, caractérisé par le fait que l'unité d'IKG contient un second additionneur analogique (24), qui est relié aux sorties de tension continue des deux redresseurs (13) commandés en phases dans les canaux (L, R) que la sortie du second additionneur analogique (24) est reliée à un second différenciateur (26) par l'intermédiaire d'un filtre passe-bande (25) à flancs abrupts, qui a une bande passante, en fonction des variations d'impédance spécifiques au cœur, comprise entre $f_u = 0,5$ Hz et $f_0 = 50$ Hz.

7. Appareil de mesure selon la revendication 6, caractérisé par le fait qu'on monte à la suite du second différenciateur (26) un détecteur de crêtes à zéro réglable, qui forme la différence entre la tension de sortie maximale du second différenciateur (26) entre deux instants de déclenchements $t_n$ et $t_{n+1}$ et la tension de sortie à l'instant de déclenchement $t_n$, qui est produit, soit intérieurement par l'intermédiaire d'un premier différenciateur (28) et d'un conformateur d'impulsions monté à sa suite, à partir de la dent R du signal EKG, soit extérieurement.

8. Appareil de mesure selon la revendication 1, caractérisé par le fait que les électrodes appliquées (1, 2, 3) sont des électrodes doubles pouvant être recouvertes d'un gel.

Fig. 1

Fig. 2

Fig. 3

$\Delta Z_{Herz}$

$\Delta$peak

$\underline{25}$

$\underline{24}$

$\underline{26}$

$\underline{27}$

$\underline{28}$

$U_{GL1}$

$U_{GL2}$

$\dfrac{dZ_{Herz}}{dt}$

EKG

Trigger/extern

intern/extern

**Fig. 4.**